(19) Europäisches Patentamt
European Patent Office
Office européen des brevets

(11) Publication number: **0 142 353 B1**

(12) **EUROPEAN PATENT SPECIFICATION**

(45) Date of publication of patent specification: **06.03.91**

(51) Int. Cl.[5]: **C01B 33/34**, B01J 29/28, C07C 5/41

(21) Application number: **84307764.5**

(22) Date of filing: **09.11.84**

The file contains technical information submitted after the application was filed and not included in this specification

(54) **High temperature zeolite L preparation.**

(30) Priority: **10.11.83 GB 8329950**

(43) Date of publication of application:
**22.05.85 Bulletin 85/21**

(45) Publication of the grant of the patent:
**06.03.91 Bulletin 91/10**

(84) Designated Contracting States:
**AT BE CH DE FR GB IT LI LU NL SE**

(56) References cited:
**EP-A- 0 096 479**
**DE-A- 2 643 928**
**GB-A- 1 202 511**

(73) Proprietor: **EXXON RESEARCH AND ENGINEERING COMPANY**
**P.O.Box 390, 180 Park Avenue**
**Florham Park, New Jersey 07932(US)**

(72) Inventor: **Verduijn, Johannes Petrus**
**Westersingel 34**
**NL-3202 XL Spijkenisse(NL)**
Inventor: **Wortel, Theodorus Maria**
**834 Halewood Drive**
**Houston Texas(US)**

(74) Representative: **Northover, Robert Frank et al**
**ESSO Chemical Limited Esso Chemical Research Centre P.O. Box 1**
**Abingdon Oxfordshire, OX13 6BB(GB)**

**EP 0 142 353 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the preparation of zeolite L and in particular it relates to the preparation of zeolite L with cylindrical morphology.

Zeolite L has been known for some time as an adsorbant, and in US 3 216 789 is described as an aluminosilicate of the formula:

0.9 - 1.3 $M_{2/n}O$: $Al_2O_3$: 5.2 - 6.9 $SiO_2$: $yH_2O$ (where M is an exchangeable cation of valence n and y is from 0 to 9) having an X-ray diffraction pattern with the following more significant d(Å) values:

16.1 ± 0.3
7.52 ± 0.04
6.00 ± 0.04
4.57 ± 0.04
4.35 ± 0.04
3.91 ± 0.02
3.47 ± 0.02
3.28 ± 0.02
3.17 ± 0.01
3.07 ± 0.01
2.91 ± 0.01
2.65 ± 0.01
2.46 ± 0.01
2.42 ± 0.01
2.19 ± 0.01

The preparation of zeolite L described in US 3 216 789 comprises crystallizing the zeolite from a reaction mixture comprising mole ratios:

| | |
|---|---|
| $K_2O/(K_2O + Na_2O)$ | 0.33 - 1 |
| $(K_2O + Na_2O)/SiO_2$ | 0.35 - 0.5 |
| $SiO_2/Al_2O_3$ | 10 - 28 |
| $H_2O/(K_2O + Na_2O)$ | 15 - 41 |

and describes crystallization temperatures of from 100°c to 150°C.

British Patent 1 202 511 describes a revised zeolite L preparation using lower proportions of silica in the reaction mixture which comprises mole ratio of reactants as:

| | |
|---|---|
| $K_2O/(K_2O + Na_2O)$ | 0.7 - 1 |
| $(K_2O + Na_2O)/SiO_2$ | 0.23 - 0.35 |
| $SiO_2/Al_2O_3$ | 6.7 - 9.5 |
| $H_2O/(K_2O + Na_2O)$ | 10.5 - 50 |

and a crystallization temperature of from 100°C to 200°C. The ratio $H_2O/(K_2O + Na_2O + SiO_2 + Al_2O_3)$ is preferably not greater than 6 to give a "dry gel".

US 3 867 512 discloses a preparation of zeolite L from a reaction mixture having a molar composition:

| | |
|---|---|
| $K_2O/(K_2O + Na_2O)$ | 0.3 - 1 |
| $(K_2O + Na_2O)/SiO_2$ | 0.3 - 0.6 |
| $SiO_2/Al_2O_3$ | 10 - 40 |
| $H_2O/(K_2O + Na_2O)$ | 15 - 140 |

in which the silica source is a gel having at least 4.5 weight percent water and prepared in a particular manner. The zeolite L is prepared at temperatures of from 140°F to 280°F (60°C to 138°C).

L Wilkosz in Pr Chem 409 (1974) - Chemical Abstracts, vol 90 (1979) 57347f describes the preparation of zeolite L from a synthesis sol prepared by treating a solution containing silica, potassium hydroxide and sodium hydroxide with a second solution containing potassium aluminate, potassium hydroxide and sodium hydroxide and crystallizing for 72 hours at 20°C and 122 hours at 100°C. The zeolite L product has a $SiO_2:Al_2O_3$ ratio of 6.4:1.

G V Tsitsishvilli et al in Doklady Akademii NaukSSSR, Vol 243, No 2, pp438-440 (1978) describe the synthesis of zeolite L from alumina-silica gels containing tributylamine. The gels used had the following molar ratios:

| | |
|---|---|
| $SiO_2:Al_2O_3$ | 25 |
| $(K_2O+Na_2O):Al_2O_3$ | 18 |
| $(K_2O+Na_2O):SiO_2$ | 0.72 |
| $H_2O/K_2O+Na_2O$ | 20 |
| $K_2O:Na_2O$ | 0.5 |

Crystallization temperatures of 90°C and 98°C were employed.

Y Nishiimura in Nippon Kagaku Zasshi 91, 11, 1970, pp 1046-9 describes in general terms zeolite L preparation from a synthesis mixture containing colloidal silica, potassium aluminate and potassium hydroxide having a $SiO_2:Al_2O_3$ ratio of 15-25 at a temperature of 100°C, but exemplifies only two synthesis mixtures having the following ratios of components:

$7K_2O:Al_2O_3:20SiO_2:450H_2O$; and

$8K_2O:Al_2O_3:10SiO_2:500H_2O$.

US 3 298 780 describes the preparation of zeolite UJ by a process in which an aqueous reactant solution having a composition, expressed as mole ratios of oxides, corresponding to

$SiO_2/Al_2O_3$ of from 6 to 30,

$R_{2/v}O/SiO_2$ of from 0.30 to 0.70, and

$H_2O/R_{2/v}O$ of from 80 to 140;

is maintained at a temperature between 150°F (65.6°C) and 325°F (162.8°C) until the zeolite crystals are formed. Zeolite UJ is described as having nearly cubic shaped crystals with a crystal size ranging upward from 0.05 micron.

GB 1 393 365 describes zeolite AG1 as being related to zeolite L and prepared by reacting at least one aluminium component, at least one silicon component and at least one alkali metal component, in an aqueous medium, the sole or major silicon component being a water glass having a molar ratio $SiO_2/M_2O$ of 3.5 to 4.0 to give a reaction mixture with oxide molar ratios in one of the following ranges:

| | | |
|---|---|---|
| Range 1 | $SiO_2/Al_2O_3$ | 7 - 14 |
| | $(K_2O+Na_2O)/SiO_2$ | 0.25 - 0.85 |
| | $K_2O/(K_2O+Na_2O)$ | 0.75 - 1.0 |
| | $H_2O/(K_2O+Na_2O)$ | 25 - 160 |
| Range 2 | $SiO_2/Al_2O_3$ | 14 - 20 |
| | $(K_2O+Na_2O)/SiO_2$ | 0.25 - 0.85 |
| | $K_2O/(K_2O+Na_2O)$ | 0.5 - 1.0 |
| | $H_2O/(K_2O+Na_2O)$ | 25 - 160 |
| Range 3 | $SiO_2/Al_2O_3$ | 20 - 40 |
| | $(K_2O+Na_2O)/SiO_2$ | 0.25 - 1.0 |
| | $K_2O/(K_2O+Na_2O)$ | 0.4 - 1.0 |
| | $H_2O/(K_2O+Na_2O)$ | 25 - 160 |

at a temperature of from 50 to 150°C.

It was subsequently found that zeolite L may be used as a catalyst base in aromatization reactions. US 4 104 320 discloses dehydrocyclization of aliphatic compounds in the presence of hydrogen using a catalyst comprising zeolite L and a group VIII metal. European Patent Application Publication 40119 discloses a dehydrocyclization process operating at low pressure (1 to 7 bars) or low $H_2$/hydrocarbon ratio using a catalyst comprising platinum on a potassium zeolite L. BE 888365 describes dehydrocyclization using a catalyst comprising platinum, rhenium (incorporated in the form of its carbonyl) and sulphur to give an atomic ratio of sulphur to platinum of 0.05 to 0.6 on a zeolitic crystalline aluminosilicate base such as zeolite L. BE 792608 discloses the treatment of zeolite L for use as catalyst in isomerization by exchange with ammonium and chromium ions.

In our copending European Patent Application Publication No. 96479 there is described and claimed a new form of zeolite material similar to conventionally prepared zeolite L, but having a characteristic morphology and size, which is particularly valuable for use as a catalyst base in hydrocarbon conversions such as aromatization. This zeolite has an X-ray diffraction (XRD) pattern obtained with CuKα radiation

having the following significant d (Å) values:

TABLE A

| |
|---|
| 16.1 ± 0.4 |
| 7.52 ± 0.05 |
| 6.00 ± 0.04 |
| 4.57 ± 0.04 |
| 4.35 ± 0.04 |
| 3.91 ± 0.02 |
| 3.47 ± 0.02 |
| 3.28 ± 0.02 |
| 3.17 ± 0.02 |
| 3.07 ± 0.02 |
| 2.91 ± 0.02 |
| 2.65 ± 0.02 |
| 2.46 ± 0.02 |
| 2.42 ± 0.01 |
| 2.19 ± 0.01 |

and comprising crystallites in the form of cylinders with a mean diameter of at least 0.1 micron, preferably at least 0.5 micron. The above XRD lines characterize the zeolite of the invention and correspond to those identified as characteristic of zeolite L in US 3 216 789. The spectrum of the zeolite will also generally show additional lines, but in general the ten most prominent peaks in the XRD pattern of the materials of the invention are given in Table B below:

TABLE B

| |
|---|
| 16.1 ± 0.4 |
| 4.57 ± 0.04 |
| 3.91 ± 0.02 |
| 3.66 ± 0.02 |
| 3.47 ± 0.02 |
| 3.28 ± 0.02 |
| 3.17 ± 0.02 |
| 3.07 ± 0.02 |
| 2.91 ± 0.02 |
| 2.65 ± 0.02 |

The positions and relative intensites of the X-ray lines are found to vary only slightly for various alkali and alkaline earth metal cation forms of the materials. However, the intensity of the line at d = 16.1 ±.3 Å has been observed to be more variable than that of other prominent lines. This is believed to be a result of the sensitivity of this peak to the preparation of the XRD sample and not to significant changes in crystal structure of the zeolite. Occasionally, additional lines not belonging to the pattern for zeolite L appear in a pattern along with the X-ray lines characteristic of the zeolite. This is an indication that one or more additional crystalline materials are mixed with zeolite L in the sample being tested. While the presence of some additional materials may be tolerated without the catalytic properties of the zeolite L being affected too adversely, it is a preferred feature of the invention that the amount of such additional crystalline materials is minimised in the zeolite material as synthesized. The synthesis of the present invention is preferably conducted so that the amount of zeolite W or denser phases in the product of the synthesis is at least reduced to lower levels - preferably so that the ratio zeolite W/zeolite L (as measured by the ratio of peak heights of lines at d = 7.09 ± 0.05 Å and d = 3.91 ± 0.03 Å in the CuKα XRD spectrum of the product) is 0.3 or less, more preferably 0.2 or less. The synthesis of the invention may be conducted such that the product of the synthesis is substantially free of any additional crystalline phase giving rise to a line in the X-ray pattern at d (Å) value of 6.28 ± 0.05 and/or 3.14 ± 0.02 .

References herein to zeolite W refer to the material described as such by D W Breck in "Zeolite Molecular Sieves", Wiley Interscience, 1974, also described as Zeolite KM in "Zeolite Technology and Application, Recent Advances", J Scott, Noyes Data Corpn., USA, 1980, p36.

The particular X-ray technique and/or apparatus employed, the humidity, the temperature, the orientation of the crystals in the sample and other variables, all of which are well known and understood to those skilled in the art of X-ray crystallography or diffraction, may also cause some variations in the positions and intensities of the X-ray lines. Thus, the X-ray data given herein to identify the zeolites prepared in the invention are not to exclude those materials which, due to some variable mentioned above or otherwise known to those skilled in the art fail to show all of the tabulated X-ray lines, or show a few extra ones that are permissible to the crystal system of the zeolite, or show a shift in position or slight change in intensity of some of the X-ray lines.

The copending European Patent Application 96479 describes the preparation of the new zeolite material as cylindrical crystallites with an aspect ratio of at least 0.5, in which an alkaline reaction mixture comprising water, a source of silicon and a source of aluminium with a composition falling within the following molar ratios (expressed as oxides):

| | |
|---|---|
| $M_2O/SiO_2$ | 0.22 - 0.36 |
| $H_2O/M_2O$ | 25 - 90 |
| $SiO_2/Al_2O_3$ | 6 - 15 |

(wherein M is a cation of valence n, and preferably potassium or a mixture of K + M' in which M' is an alkali metal or alkaline earth metal such as sodium, calcium, barium, or rubidium, provided that $K_2O/(M'_2O + K_2O)$ is at least 0.7) is heated to a temperature of from at least 75°C and preferably from about 100°C to about 250°C, more preferably from about 120°C to about 225°C, to form the desired cylindrical aluminosilicate.

It has now been found that zeolite L may be obtained from crystallization gels with relatively low water contents by employing higher crystallization temperatures and shorter crystallization times, and that such high temperature process give improved yields of zeolite L. Further the zeolite L thus produced has demonstrated excellent activity as a catalyst base in aromatization.

Thus in one aspect this invention provides a process for the preparation of zeolite L in which an alkaline reaction mixture comprising water, a source of silicon and a source of aluminium with a composition having the following molar ratio (expressed as oxides):

| | |
|---|---|
| $M_2O/SiO_2$ | 0.24 - 0.30 |
| $H_2O/M_2O$ | 25 - 45 |
| $SiO_2/Al_2O_3$ | 10 - 12 |

(wherein M is a monovalent cation is heated to a temperature of greater than 150°C for from 5 to 24 hours to form Zeolite L. It is particularly surprising that operating at higher temperatures highly crystalline forms of Zeolite L may be obtained. In a further aspect the invention provides a process in which the reaction mixture has a composition with the following molar ratio (expressed as oxides):

| | |
|---|---|
| $M_2O/SiO_2$ | 0.24 - 0.30 |
| $H_2O/M_2O$ | 25 - 45 |
| $SiO_2/Al_2O_3$ | 7 - 12 |

(wherein M is a monovalent cation which is heated to a temperature of greater than 200°C for less than 24 hours to form zeolite L, the crystallization being preferably discontinued prior to the transformation of

significant amounts of the formed zeolite to other crystalline phases giving lines d = 6.28 $\pm$ 0.05 Å and 3.14 $\pm$ 0.01 Å in the XRD spectrum.

It has been found that good yields of zeolite L may be obtained by the processes of the invention, and that products may be prepared similar to those shown in our copending European Application Publication No 96479 to provide an excellent catalyst base for hydrocarbon conversions and particularly aromatization reactions. Surprisingly, the products of the present invention have shown the capability of providing catalysts with longer lifetime than the products of our copending Application.

It is a further surprising feature of the present invention that Zeolite L may be formed with relatively dry gels at high temperatures. The ratio of $H_2O/(M_2O + SiO_2 + Al_2O_3)$ is preferably less than 8 and may be less than 6. In combination with the lower water content the invention places a lower limit on the $M_2O/SiO_2$ ratio and it is the combination of the drier gels with lower $K_2O$ content that enables the desired product to be obtained at higher temperatures. Also drier gels may make the reaction mixture easier to handle being in the form of a semi-solid rather than a slurry. However, while dry gels are described in GB 1 202 511 the same requirement for reduced $K_2O$ content is not appreciated. GB 1 202 511 does not give good yields of zeolite L, as shown hereinafter, we have found that by operating at higher temperatures highly crystalline Zeolite L may be obtained with cylindrical morphology.

The dry yields (i.e. the yield obtained after fully drying the product - for example, by heating to 150 °C for 16 hours) obtained by the process of the invention are typically greater than 16 wt% (based on the weight of the reaction mixture), and better yields appear to be obtained with lower water contents. Yields of greater than 18%, or even greater than 20% may be obtained, and as shown in the Examples yields of greater than 24% have been achieved. Typical yields for the process of European Application Publication No 96479 are less than 16%.

There are four principal components to the reaction mixture or synthesis gel and thus generally:

aluminium
silicon
potassium (optionally with up to 30 mole% replaced by alkali or alkaline earth metal)
water

and the relative proportions of these four components and the chosen reaction conditions must be selected within the ranges specified above to obtain good yields of zeolite L. The proportions of the four components can be shown graphically in a series of four triangular graphs each showing the percentage composition of three components of the reaction with the content of the fourth component being excluded from each graph. As described in more detail hereinafter Figures 1 to 4 show such plots for preferred reaction mixtures according to the process of the invention and, by way of comparison, the Figures also show the preferred reaction mixtures described in European Patent Application Publication No 96479 while the graphs show reaction mixtures in which M is potassium alone, it is to be understood that part of the potassium may be replaced by another alkali or alkaline earth metal it may be determined empirically whether this substitution affects the product and the graphs are to be interpreted with that in mind.

The four graphs together may be considered as the four faces of a three-dimensional graph in the form of a regular tetrahedron, and the points and areas marked thereon represent the projection on those faces of points and volumes within the three-dimensional graph. Thus, while a point may lie within the area designated as constituting the invention in one graph, unless it lies within the area of the invention on all four graphs it does not lie within the volume defined within the three-dimensional graph as being a preferred reaction mixture of the invention.

Figure 1 is a triangular graph of the content of the reaction mixture (in weight percentage of oxides excluding alumina) showing the relative proportions of water, silicon and potassium. Preferred reaction mixtures of the invention lie within the area marked $I_B$ in the graph, while the area marked $I_A$ in the graph demonstrates the preferred reaction mixtures described in EP 96479.

Figure 2 is a triangular graph of the content of the reaction mixture (in weight percent of oxides excluding water) showing the relative properties of aluminium, silicon and potassium. Preferred reaction mixtures of the invention lie within the area marked $II_B$ in the graph, while the area marked $II_A$ in the graph denotes the preferred reaction mixtures described in EP 96479.

Figure 3 is a triangular graph of the content of the reaction mixture (in weight percent of oxides excluding silica) showing the relative properties of aluminium, water and potassium. Preferred reaction mixtures of the invention lie within the area marked $III_B$ in the graph, and area $III_A$ denotes the disclosure of EP 96479.

Figure 4 is a triangular graph of the content of the reaction mixture (in weight percent of oxides

excluding potassium oxide) showing the relative properties of aluminium, silicon and water. Preferred reaction mixtures of the invention lie within the area marked $IV_B$ in the graph, and area $IV_A$ denotes the disclosure of EP 96479.

In one preferred embodiment of the invention the reaction mixture comprises the following range of molar ratios:

| | |
|---|---|
| $K_2O/SiO_2$ | 0.24 - 0.30 |
| $H_2O/K_2O$ | 25 - 35 |
| $SiO_2/Al_2O_3$ | 7 - 12 (when T>200°C)<br>10 - 12 (when T>150°C) |

In a particularly preferred embodiment an optimum composition for a process in which the reaction mixture is heated to from 200°C to 250°C to give highly crystalline Zeolite L has been found to be substantially in the following range of mole ratios:
$2.6\ K_2O:Al_2O_3:10SiO_2:70\text{-}120H_2O$ and particularly good results have been obtained by heating to about 225°C. As demonstrated hereinafter a reaction mixture of this type with a water content of 80 moles if subjected to a crystallization temperature of 150°C does not yield cylindrical zeolite L. However, by employing a high enough crystallization temperature a water content of even 70 moles will yield a more cylindrical form of zeolite L.

In addition to varying the proportions of the reactants in the reaction mixture it is possible to vary the reaction conditions and in particular the crystallization time and temperature. Within the reactant ratios defined for the process of the invention a lower silicon content and/or lower water content to be lowered and/or higher potassium content (and thus the alkalinity) will generally produce better yields of zeolite L at higher crystallization temperatures - 225°C or higher. Thus, the desired zeolite L may be obtained with a gel containing only 70 moles of water by crystallizing at 225°C. By contrast it will generally be possible to operate at 200°C to 225°C when higher water contents within the specified range are employed.

The crystallization time is critical in the present invention. The high temperatures employed are found to result in rapid formation of zeolite L, which has the advantage of reducing the normally lengthy crystallization time. There is, however, a tendency for other crystalline materials to be formed if the formed zeolite L is held at the crystallization temperature for too long. In particular there is a tendency to form crystalline materials which gives rise to lines in the XRD pattern (CuK∝) at 6.28 ± 0.05 Å and/or 3.14 ± 0.02 Å.

Thus the crystallization is discontinued after the previously defined time limits before there is substantial formation of such additional crystalline materials. Thus times of less than 24 hours, and generally of 5 to 15 hours will be appropriate.

The zeolites, formed in the process of the invention are preferably aluminosilicates and are described herein in terms of aluminosilicates, though other elemental substitutions are possible, for example aluminium may be substituted by gallium, boron, iron and similar di-or trivalent elements capable of existing in tetrahedral coordnation, and silicon may be substituted by elements such as germanium or phosphorus. The aluminosilicates preferably have a composition (expressed in terms of molar ratios of the constituent oxides in anhydrous form) of:

$$(0.9 - 1.3)\ M_{2/n}O:\ Al_2O_3:\ xSiO_2 \qquad (I)$$

wherein M is a cation of valence n, x is from 5 to 7.5, preferably from 5.7 to 7.4. The products of the process of the invention have high crystallinity as shown by a well-defined X-ray diffraction pattern (without binder or other diluents present) with sharp peaks. Crystallinity may be measured relative to a quartz standard by comparing the peak areas for the reflection from the 220 plane (d = 4.57 ± 0.04Å) and the 221 plane (d = 3.91 ± 0.02) for the zeolitic material of the invention with the peak area for the reflection from the 110 plane (d = 2.46 ± 0.02) of the quartz. The ratio of the combined peak areas of the 220 and 221 reflections of the zeolitic material to the peak area of the 110 reflection of quartz is a measure of the crystallinity of the sample. To provide a comparison between different samples and to eliminate cation effects the peak area determination is preferably carried out on the same cation form of the zeolite.

The exchangeable cation M in general formula I is very preferably potassium, but it is possible for a part of M to be replaced by other cations such as alkali and alkaline earth metals for example sodium, rubidium or caesium. The ratio $M_{2/n}O:Al_2O_3$ is preferably from 0.95 to 1.15, and generally above 1.

In general formula I the symbol x (the mole ratio $SiO_2:Al_2O_3$) more preferably represents from 6 to 7 and most preferably from 6.0 to 6.5.

The aluminosilicate forms of the invention may be hydrated, typically with up to 9 moles of water per mole of $Al_2O_3$. When used as a catalyst base, as described hereinafter, the zeolite of the invention is preferably first calcined to remove water. In normal preparation from aqueous gels a hydrated form is first prepared and this may be dehydrated by heating.

Scanning electron micrographs (SEM) of the materials of the invention show these to have a distinct crystal morphology. The preferred products of the process of the invention appear as to be generally in the form of cylinders in scanning electron micrographs. The terms "cylinder" and "cylindrical" are used herein to describe particles having substantially the shape of a cylinder as defined in solid geometry - that is, a solid bounded by a surface generated by a line moving parallel to a fixed line so as to cut a fixed plane curve and by two parallel planes (bases) which cut the surface. The use of these terms is not intended to exclude particles having minor surface irregularities or displaying typical crystallographic faults or dislocations. In particular, the products of the invention may show roughness on the basal planes of the cylinders, or corrugations on the lateral planes. X-ray diffraction shows the products to have a high degree of crystallinity inherent to the distinctive morphology and which is believed to result from larger domains within the crystallites. The cylindrical particles of the invention are preferably substantially in the form of circular cylinders (circular cross-section) and most preferably substantially in the form of right circular cylinders (wherein the bases are normal to the cylinder axis). Particularly preferred cylindrical particles are those having an aspect ratio (the length of the cylinder surface to the diameter of the cylinder) of at least 0.5, and more preferably at least 0.8. Particles prepared according to the invention have been shown to have excellent properties of extending catalyst life when used as catalyst bases for aromatization catalysts. This is in contrast to other morphologies, and in particular particles with cylindrical morphology have been shown better than particles with a clam-like shape. The term "clam" is used to describe particles having two generally convex faces joined to give the appearance of a clam shell. The products of the process of the invention are preferably characterized by at least 50%, more preferably 70% and most preferably 85%, of the crystallites being cylinders. The aspect ratio of the cylindrical crystallites is preferably from 0.8 to 1.5.

A further particularly surprising feature of the invention is that large crystallites wherein the mean diameter of the cylinders is at least 0.1 micron may be prepared. The cylindrical particles preferably have a mean diameter of at least 0.5 micron, more preferably of at least 0.75 micron and most preferably at least 0.9 micron. The crystallites will generally be in the range of 0.8 to 4 micron, and within that range sizes of at least 1.0 micron and/or not more than 3.0 micron are preferred.

In the synthesis of the invention, the source of silicon for the reaction mixture is generally silica, and this is usually most conveniently in the form of a colloidal suspension of silica such as Ludox HS 40 available from E.I. Dupont de Nemours and Co. ("Ludox" is a registered Trade Mark) Colloidal silicon sols are preferred since they result in less contaminating phases. However other forms such as silicates may be used.

The source of aluminium may be an alumina introduced into the reaction medium as, for example, $Al_2O_3.3H_2O$, previously dissolved in alkali. However, it is also possible to introduce aluminium in the form of the metal, which is dissolved in alkali.

The process of the invention preferably uses a reaction mixture containing potassium. This potassium is preferably introduced as potassium hydroxide. The reaction mixture may contain small quantities of other metal cations and salt forming anions as already described, but it has been found that there is an increasing tendency for other aluminosilicates to be found as the content of other ions is increased, resulting in less pure forms of the aluminosilicate of the invention. For example, sodium and rubidium ions favour erionite formation, caesium ions favour pollucite formation. Thus it is preferred for potassium hydroxide to be the source of potassium and the source of alkalinity.

The product of the processes described above is predominantly a potassium form of the aluminosilicate - that is, aluminosilicate where M in general formula I is K. By ion exchange of the product in the manner conventional to zeolite chemistry other cations such as Na or H can be introduced for M.

Within the ranges specified hereinbefore for the composition of the reaction mixture it is possible to choose ratios of oxides and alkalinity to given particular forms of the aluminosilicate product. The $SiO_2/Al_2O_3$ ratio in the product preferably lies in a relatively narrow range of 5.7 to 7.4. The higher the $SiO_2/Al_2O_3$ ratio in the reaction mixture, the higher the ratio in the product. Also, decreasing alkalinity ($OH^-/SiO_2$) tends to increase the $SiO_2/Al_2O_3$ ratio in the formed product. Dilution of the reaction mixture with

water and thus increasing the $H_2O/K_2O$ ratio also tends to increase the $SiO_2/Al_2O_3$ ratio in the product. Other cations such as tetramethylammonium and the presence of other potassium salts can be used to raise the $SiO_2/Al_2O_3$ ratio, but as described hereinbefore this may also result in the formation of other zeolite forms.

The crystallization is generally carried out in a sealed autoclave and thus at autogenous pressure. Lower pressure will require longer crystallization times and will tend to decrease the aspect ratio of the crystallites.

Following the preparation as described above the formed zeolite may be separated, washed and dried in the normal manner.

The products of the processes of the invention described hereinbefore are preferably substantially free from contaminant crystalline and amorphous materials. However, in employing these products in catalytic applications it may be desired to combine them with additional crystalline or amorphous materials and this invention extends to such combinations.

We have found that the products of the invention are excellent catalyst bases and may be used in a wide variety of catalytic reaction. The particular morphology of the crystals appears to result in a particular stable base for catalytically active metals with a surprising resistance to metal catalyst deactivation. In addition, their low acidity which makes them especially suited to catalytic applications where a low acid site strength is advantageous such as aromatization.

The catalytically-active metal(s) may be, for example, a Group VIII metal such as platinum, tin, or germanium as described in US 4 104 320, or a combination of platinum and rhenium as described in GB 2 004 764 or BE 888365. In the latter case the catalyst may for appropriate circumstances also incorporate halogen as described in US 4 165 276, silver as described in US 4 295 959 and US 4 206 040, cadmium as described in US 4 295 960 and US 4 231 897 or sulphur as described in GB 1 600 927.

We have found a particularly advantageous catalyst composition to incorporate from 0.1 to 6.0 weight %, preferably from 0.1 to 1.5 weight % platinum or palladium, since this gives excellent results in aromatization. From 0.4 to 1.2 wt % platinum is particularly preferred, especially in conjuction with the potassium form of the aluminosilicate.

It may also be useful to incorporate into the catalyst one or more materials substantially inert under the conditions in which the catalyst is to be employed to act as a binder. Such binders may also act to improve the resistance of the catalyst to temperature, pressure and attrition.

The products of the invention may be used in a process for the conversion of a hydrocarbon feed in which the feed is contacted with a catalyst as described above under appropriate conditions to bring about the desired conversion. They may for example be useful in reactions involving aromatization and/or dehydrocyclization and/or isomerization and/or dehydrogenation reaction. They are particularly useful in a process for the dehydrocyclization and/or isomerization of acyclic hydrocarbons in which the hydrocarbons are contacted at a temperature of from 430 °C to 550 °C with a catalyst comprising an aluminosilicate of the invention having at least 90% of the exchangeable cations M as alkali metal ions and incorporating at least one Group VIII metal having dehydrogenating activity, so as to convert at least part of the acyclic hydrocarbons into aromatic hydrocarbons.

The process is preferably otherwise carried out in the manner described in US 4 104 320, BE 888365 or EP 40119.

It has been found that use of the aluminosilicates of the invention in this way enables greatly improved catalyst lifetimes to be achieved as compared to the lifetime obtained with a conventionally prepared zeolite.

The invention will now be described in more detail, though only by way of illustration, in the following Examples and Evaluations, with reference to the accompanying drawings, in which:

Figures 1 to 4 are, as described hereinbefore, triangular graphs showing the preferred proportions of components of the synthesis gel. On these graphs the reaction mixtures used in the Examples in the production of aluminosilicates of the invention are shown as crosses identified by the number of Example, and those reaction mixtures used in the Comparative Examples are shown as solid circles identified by the letter of the Comparative Example. It is pointed out that the points marked for Example 3 and Comparative Example D coincide and are marked only with a cross.

## Example 1: Preparation of Zeolite L

A synthesis gel was prepared having the following composition expressed in moles of pure oxide:

$2.6\ K_2O:Al_2O_3:10SiO_2:101H_2O$

This gel was prepared as follows:
9.76g of aluminium hydroxide was dissolved by boiling in an aqueous solution of 20.92g of potassium hydroxide pellets (86% pure KOH) in 32.55g of water to form solution A. After dissolution any water loss was corrected. A separate solution, solution B, was prepared by diluting a 94.15g of colloidal silica (Ludox HS40) with 18.25g of water.

Solutions A and B were mixed for two minutes to form 173.41g of gel, and just before the gel became fully stiff it was transferred to a Teflon-lined 150ml autoclave, which was transferred to an oven at 225° C. ("Teflon" is a registered Trade Mark). The gel reached 225° C in about 4 hours. The gel was held at that temperature to bring about crystallization - the total heat-up and crystallization time being 8 hours.

34.5g of Zeolite L were formed (19.9% yield) and this was highly crystalline with a typical Zeolite L X-ray diffraction (XRD) pattern. Scanning electron micrographs (SEM) show the product to be formed solely of generally cylindrical crystals with rough lateral planes having a particle size of 0.5 to 1 microns and an aspect ratio of 1. No zeolite W or crystalline non-zeolitic phases were detectable in the XRD pattern.

Examples 2-8 and Comparative Examples A and B:

Variation in water content/crystallization condition

The general procedure of Example 1 was repeated using reduced amounts of water in solution A and without additional water in solution B. The mole ratios of the reactants are given in Table 1 below together with details of the products obtained. Various reaction temperatures and times were investigated.

To provide a comparison in Comparative Example A a low water synthesis was carried out at 150° C and a clam shaped Zeolite L product was obtained, which has been shown in copending European Application No. EP-A-96 479 to have poor catalytic performance. Comparision Example B shows the poorer yield obtained with a process as described in the co-pending Application.

Table 1

| Example | Gel wt(g) | Mole ratio reactants | Temperature (°C)/ Time (hr) | Product wt(g) | Yield (%) | Particle size (u) | Particle shape | Aspect Ratio |
|---|---|---|---|---|---|---|---|---|
| 1 | 173.41 | $2.6K_2O:Al_2O_3:10SiO_2:101H_2O$ | 225/8 | 34.5 | 19.9 | 0.5-1 | cylinder | 1 |
| 2 | 193.52 | $2.6K_2O:Al_2O_3:10SiO_2:90H_2O$ | 225/8 | 40.2 | 20.8 | 1-2 | cylinder | 0.5-1 |
| 3 | 176.80 | $2.6K_2O:Al_2O_3:10SiO_2:80H_2O$ | 225/8 | 39.4 | 22.3 | 1-2 | cylinder | 0.5-1 |
| 4 | 172.7 | $2.6K_2O:Al_2O_3:10SiO_2:70H_2O$ | 225/16 | 43.0 | 24.9 | 1-2 | rough cylinders[1] | 0.5 |
| 5 | 210.23 | $2.6K_2O:Al_2O_3:10SiO_2:100H_2O$ | 200/16 | 42.0 | 20.0 | 1-2 | cylinder | |
| 6 | 196.45 | $2.6K_2O:Al_2O_3:10SiO_2:90H_2O$ | 200/16 | 41.9 | 21.3 | 1-2 | rough cylinder | 0.4-1 |
| 7 | 182.82 | $2.6K_2O:Al_2O_3:10SiO_2:80H_2O$ | 200/16 | 41.8 | 22.9 | 1-2 | rough cylinder | 0.4-1 |
| 8 | 169.45 | $2.6K_2O:Al_2O_3:10SiO_2:70H_2O$ | 200/16 | 39.5 | 24.7 | 1-3 | clam and cylinder, trace of denser phase | 0.3 |
| A | | $2.6K_2O:Al_2O_3:10SiO_2:80H_2O$ | 150/72 | - | - | 1.5-2 | clams | - |
| B | | $2.6K_2O:Al_2O_3:10SiO_2:160H_2O$ | 150/72 | - | 15.4 | 1-1.5 | cylinders | 1 |

Note 1: Some denser phase present, repeating this experiment with a crystallization time reduced to 5 hours resulting in à product containing no denser phase

Comparative Example C

Repetition of DT 1813099 Example 6

A synthesis gel was prepared having substantially the composition described in Example 6 of DT 1813099 (equivalent to GB 1 202 511):

$2.75K_2O:Al_2O_3:8.7SiO_2:100H_2O$ 7.37g of aluminium hydroxide were dissolved in an aqueous solution of 16.98gms of potassium hydroxide (86% pure KOH) in 30.9gms water to Solution A. 25.04g of silica as Aerosil 200 were mixed with 55.4g water for 5 minutes to form Solution B. ("Aerosil is a registered Trade Mark) Solutions A and B were mixed for 1 minute and the formed putty-like gel was heated in an autoclave at 140°C for 46.5 hours.

The product was separated and dried as in Example 1. XRD and SEM showed the product to be a mixture of Zeolite W with zeolite L. No cylindrical crystallites were observed. The procedure of DT 1813099 using drying gels and lower crystallization temperatures than the present invention did not result in the highly crystalline zeolite L formed by the invention.

Evaluation: Aromatization

The performance of the Zeolite L sample of the Example 3 as a catalyst base in aromatization was determined. A catalyst was prepared by impregnating the base with 0.6 wt% platinum.

The zeolite sample under test (typically about 25g) was slurried in 700ml of distilled water in a 5 litre flask. The appropriate amount of tetraammino-platinum dichloride to give impregnation at a rate of 0.6wt% platinum was dissolved in 300ml of distilled water. The platinum salt solution was then added to the sample slurry over an 6 hour period. The mixture was stirred for 24 hours after platinum salt addition, then the impregnated sample was dried at 110°C.

The sample was then pelletized, crushed to 14-20 mesh (US sieve size) and loaded into a vertical tubular reactor and air (substantially water-free) was passed over the catalyst at a rate of 25ml/min of air per gram of sample. The catalyst was heated to 480°C in four hours and held at 480°C for three hours. The sample was then reduced by passing hydrogen over it at 207kPa and a rate of 75ml/min per gram of sample, while the catalyst was heated to 527°C in 3 hours, held at 527°C for ten minutes and then cooled to the desired reaction temperature.

The aromatization test was carried out at a temperature of 510°C and 690 KPa (100 psig) pressure with a $C_6$ mixed feed comprising:

| Component | wt% |
|---|---|
| iso-$C_6$ (3-methyl-pentane) | 30 |
| n - $C_6$ | 60 |
| methyl cyclopentane | 10 |

at a space velocity of 2.5 wt/wt/hr and in the presence of hydrogen, the $H_2$: hydrocarbon ratio being 6.

The results are given in Table 2, which shows the performance of the catalyst after 20,146 and 265 hours. On the basis of the rate of deactivation over the period investigated, it is estimated that the catalyst would give an average of 50% benzene yield over a period of 1140 hours.

Table 2

| Results (wt%) | Hours on test 20 | 146 | 265 |
|---|---|---|---|
| $C_6$ conversion | 96.8 | 90.3 | 84.6 |
| Benzene yield | 67.2 | 64.4 | 59.7 |
| Benzene selectivity | 69.4 | 71.4 | 70.6 |
| $C_4^-$ yield | 16.0 | 11.7 | 10.5 |

## Claims

1. A process for the preparation of zeolite L in which an alkaline reaction mixture comprising water, a source of silicon and a source of aluminium with a composition having the following molar ratio (expressed as oxides)

| | |
|---|---|
| $M_2O/SiO_2$ | 0.24 - 0.30 |
| $H_2O/M_2O$ | 25 - 45 |
| $SiO_2/Al_2O_3$ | 10 - 12 |

(wherein M is a monovalent cation) is heated to a temperature of greater than 150° C for from 5 to 24 hours to form zeolite L.

2. A process for the preparation of zeolite L in which an alkaline reaction mixture comprising water, a source of silicon and a source of aluminium with a composition having the following molar ratio (expressed as oxides):

| | |
|---|---|
| $M_2O/SiO_2$ | 0.24 - 0.30 |
| $H_2O/M_2O$ | 25 - 45 |
| $SiO_2/Al_2O_3$ | 7 - 12 |

(wherein M is a monovalent cation) is heated to a temperature of greater than 200° C for less than 24 hours to form zeolite L.

3. A process as claimed in claim 2, in which the mole ratio of $H_2O/(M_2O + SiO_2 + Al_2O_3)$ is less than 8.

4. A process as claimed in claim 3, in which the mole ratio of $H_2O/(M_2O + SiO_2 + Al_2O_3)$ is less than 6.

5. A process as claimed in any of claims 2-4, in which the reaction mixture is such that it lies within the area marked $I_B$ in the graph of Figure 1, within the area marked $II_B$ in the graph of Figure 2, within the

area marked $III_B$ in the graph of Figure 3 and within the area marked $IV_B$ in the graph of Figure 4.

6. A process as claimed in any of claims 2-5, in which the reaction mixture has the following molar ratios:

| | |
|---|---|
| $K_2O/SiO_2$ | 0.24 - 0.30 |
| $H_2O/K_2O$ | 25 - 35 |
| $SiO_2/Al_2O_3$ | 7 - 12 |

7. A process as claimed in any of claims 2 to 5, in which the reaction mixture has substantially the following range of mole ratios:
   2.6 $K_2O$:$Al_2O_3$:10$SiO_2$:70-120$H_2O$

8. A process as claimed in claim 7, in which the reaction mixture is heated to from 200°C to 250°C.

9. A process as claimed in any of claims 2 to 8 in which the crystallization is discontinued prior to the transformation of significant amounts of zeolite L to other crystalline phases.

10. A process as claimed in claim 9, in which the crystallization time is 5 to 15 hours.

11. A catalyst comprising a zeolite L obtainable by a process as claimed in any of claims 1 to 10 and a catalytically-active metal.

**Revendications**

1. Procédé de préparation de zéolite L, dans lequel un mélange réactionnel alcalin comprenant de l'eau, une source de silicium et une source d'aluminium ayant une composition correspondant aux rapports molaires suivants (exprimés sur la base des oxydes) :

| | |
|---|---|
| $M_2O/SiO_2$ | 0,24 - 0,30 |
| $H_2O/M_2O$ | 25 - 45 |
| $SiO_2/Al_2O_3$ | 10 - 12 |

(dans lesquels M représente un cation monovalent) est chauffé à une température supérieure à 150°C pendant un temps de 5 à 24 heures pour former la zéolite L.

2. Procédé de préparation de zéolite L, dans lequel un mélange réactionnel alcalin comprenant de l'eau, une source de silicium et une source d'aluminium ayant une composition correspondant aux rapports molaires suivants (exprimés sur la base des oxydes) :

| | |
|---|---|
| $M_2O/SiO_2$ | 0,24 - 0,30 |
| $H_2O/M_2O$ | 25 - 45 |
| $SiO_2/Al_2O_3$ | 7 - 12 |

(dans lesquels M représente un cation monovalent) est chauffé à une température supérieure à 200°C pendant un temps inférieur à 24 heures pour former la zéolite L.

3. Procédé suivant la revendication 2, dans lequel le rapport molaire $H_2O/(M_2O+SiO_2Al_2O_3)$ est inférieur à 8.

4. Procédé suivant la revendication 3, dans lequel le rapport molaire $H_2O/(M_2O+SiO_2+Al_2O_3)$ est

inférieur à 6.

5. Procédé suivant l'une quelconque des revendications 2 à 4, dans lequel le mélange réactionnel est choisi de manière qu'il se trouve dans la surface désignée par $I_B$ sur le graphique de la Figure 1, à l'intérieur de la surface désignée par $II_B$ sur le graphique de la Figure 2, à l'intérieur de la surface désignée par $III_B$ sur le graphique de la Figure 3 et à l'intérieur de la surface désignée par $IV_B$ sur le graphique de la Figure 4.

6. Procédé suivant l'une quelconque des revendications 2 à 5, dans lequel le mélange réactionnel possède les rapports molaires suivants :

| | |
|---|---|
| $K_2O/SiO_2$ | 0,24 - 0,30 |
| $H_2O/K_2O$ | 25 - 35 |
| $SiO_2/Al_2O_3$ | 7 - 12 |

7. Procédé suivant l'une quelconque des revendications 2 à 5, dans lequel le mélange réactionnel correspond pratiquement à l'intervalle de rapports molaires suivant :
$2{,}6\ K_2O:Al_2O_3:10SiO_2:70\text{-}120H_2O$

8. Procédé suivant la revendication 7, dans lequel le mélange réactionnel est chauffé à une température de 200 °C à 250 °C.

9. Procédé suivant l'une quelconque des revendications 2 à 8, dans lequel la cristallisation est interrompue avant la transformation de quantités importantes de zéolite L en d'autres phases cristallines.

10. Procédé suivant la revendication 9, dans lequel le temps de cristallisation est compris dans l'intervalle de 5 à 15 heures.

11. Catalyseur comprenant une zéolite L pouvant être obtenue par un procédé suivant l'une quelconque des revendications 1 à 10 et un métal catalytiquement actif.

**Ansprüche**

1. Verfahren zur Herstellung von Zeolith L, bei dem eine Wasser, eine Siliciumquelle und eine Aluminiumquelle umfassende, alkalische Reaktionsmischung mit einer die folgenden Molverhältnisse (ausgedrückt als Oxide) aufweisenden Zusammensetzung:

| | |
|---|---|
| $M_2O/SiO_2$ | 0,24 - 0,30 |
| $H_2O/M_2O$ | 25 - 45 |
| $SiO_2/Al_2O_3$ | 10 - 12 |

(wobei M ein einwertiges Kation ist) zur Bildung von Zeolith L 5 bis 24 Stunden auf eine Temperatur von mehr als 150 °C erhitzt wird.

2. Verfahren zur Herstellung von Zeolith L, bei dem eine Wasser, eine Siliciumquelle und eine Aluminiumquelle umfassende, alkalische Reaktionsmischung mit einer die folgenden Molverhältnisse (ausgedrückt als Oxide) aufweisenden Zusamensetzung:

| | |
|---|---|
| $M_2O/SiO_2$ | 0,24 - 0,30 |
| $H_2O/M_2O$ | 25 - 45 |
| $SiO_2/Al_2O_3$ | 7 - 12 |

(wobei M ein einwertiges Kation ist) zur Bildung von Zeolith L für weniger als 24 Stunden auf eine Temperatur von mehr als 200° C erhitzt wird.

3. Verfahren nach Anspruch 2, bei dem das Molverhältnis von $H_2O/(M_2O + SiO_2 + Al_2O_3)$ weniger als 8 beträgt.

4. Verfahren nach Anspruch 3, bei dem das Molverhältnis von $H_2O/(M_2O + SiO_2 + Al_2O_3)$ weniger als 6 beträgt.

5. Verfahren nach einem der Ansprüche 2 bis 4, bei dem die Reaktionsmischung so ist, daß sie innerhalb des mit $I_B$ markierten Bereiches in der Graphik von Figur 1, innerhalb des mit $II_B$ markierten Bereiches in der Graphik von Figur 2, innerhalb des mit $III_B$ markierten Bereiches in der Graphik von Figur 3 und innerhalb des mit $IV_B$ markierten Bereiches in der Graphik von Figur 4 liegt.

6. Verfahren nach einem der Ansprüche 2 bis 5, bei dem die Reaktionsmischung folgende molare Verhältnisse aufweist:

| | |
|---|---|
| $K_2O/SiO_2$ | 0,24 - 0,30 |
| $H_2O/K_2O$ | 25 - 35 |
| $SiO_2/Al_2O_3$ | 7 - 12. |

7. Verfahren nach einem der Ansprüche 2 bis 5, bei dem die Reaktionsmischung im wesentlichen folgenden Bereich an Molverhältnissen aufweist:
   2,6 $K_2O$ : $Al_2O_3$ : 10 $SiO_2$ : 70-120 $H_2O$

8. Verfahren nach Anspruch 7, bei dem die Reaktionsmischung auf 200 bis 250° C erhitzt wird.

9. Verfahren nach einem der Ansprüche 2 bis 8, bei dem die Kristallisation vor der Umwandlung von signifikanten Mengen an Zeolith L in andere kristalline Phasen unterbrochen wird.

10. Verfahren nach Anspruch 9, bei dem die Kristallisationszeit 5 bis 15 Stunden beträgt.

11. Katalysator, der einen Zeolith L erhalten nach einem Verfahren gemäß einem der Ansprüche 1 bis 10 und ein katalytisch aktives Metall enthält.

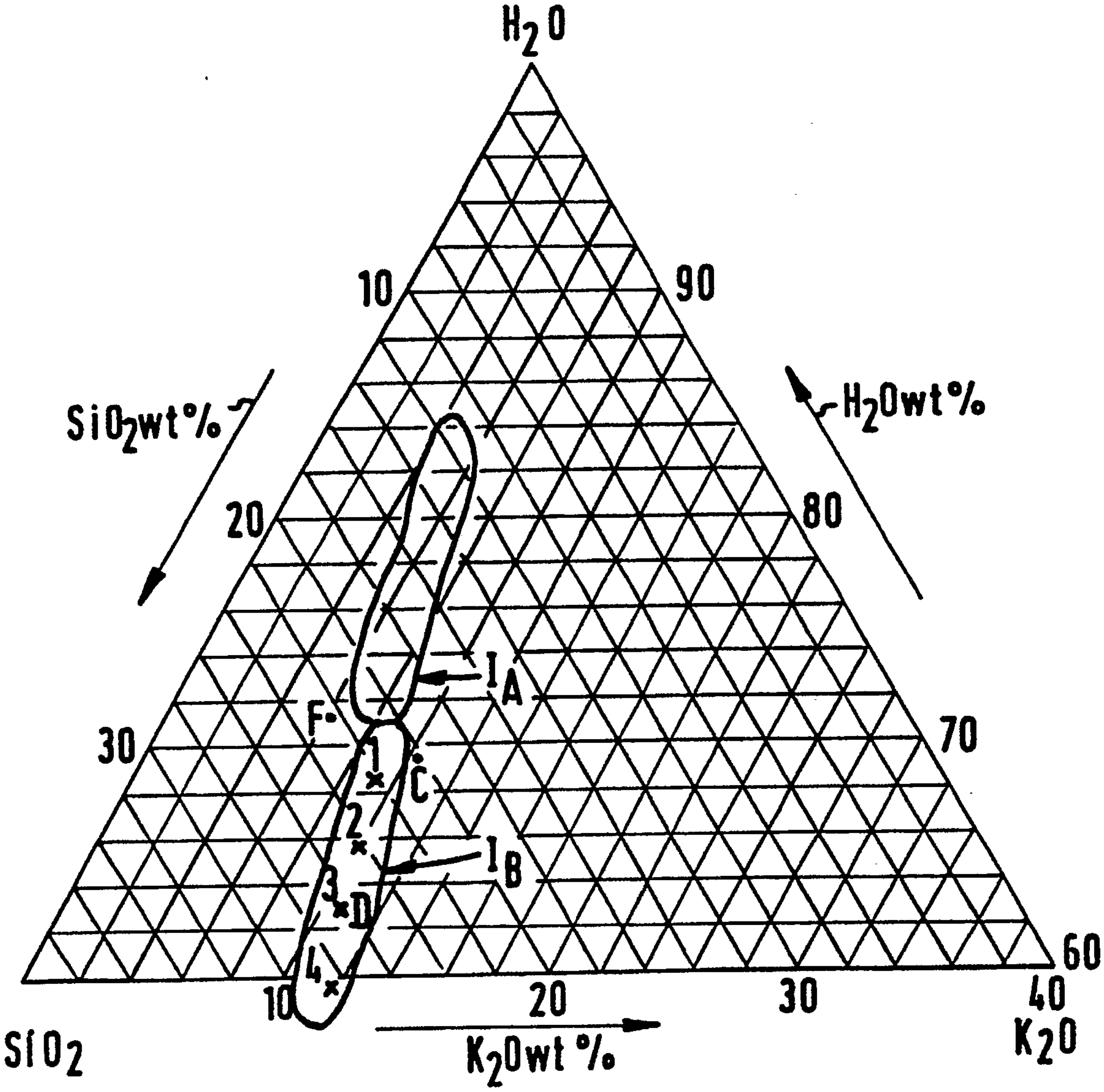
H2O
10
90
SiO2wt%
H2Owt%
20
80
IA
F
30
70
1
C
2
IB
3
D
4
60
10
20
30
40
SiO2
K2Owt%
K2O

FIG.1

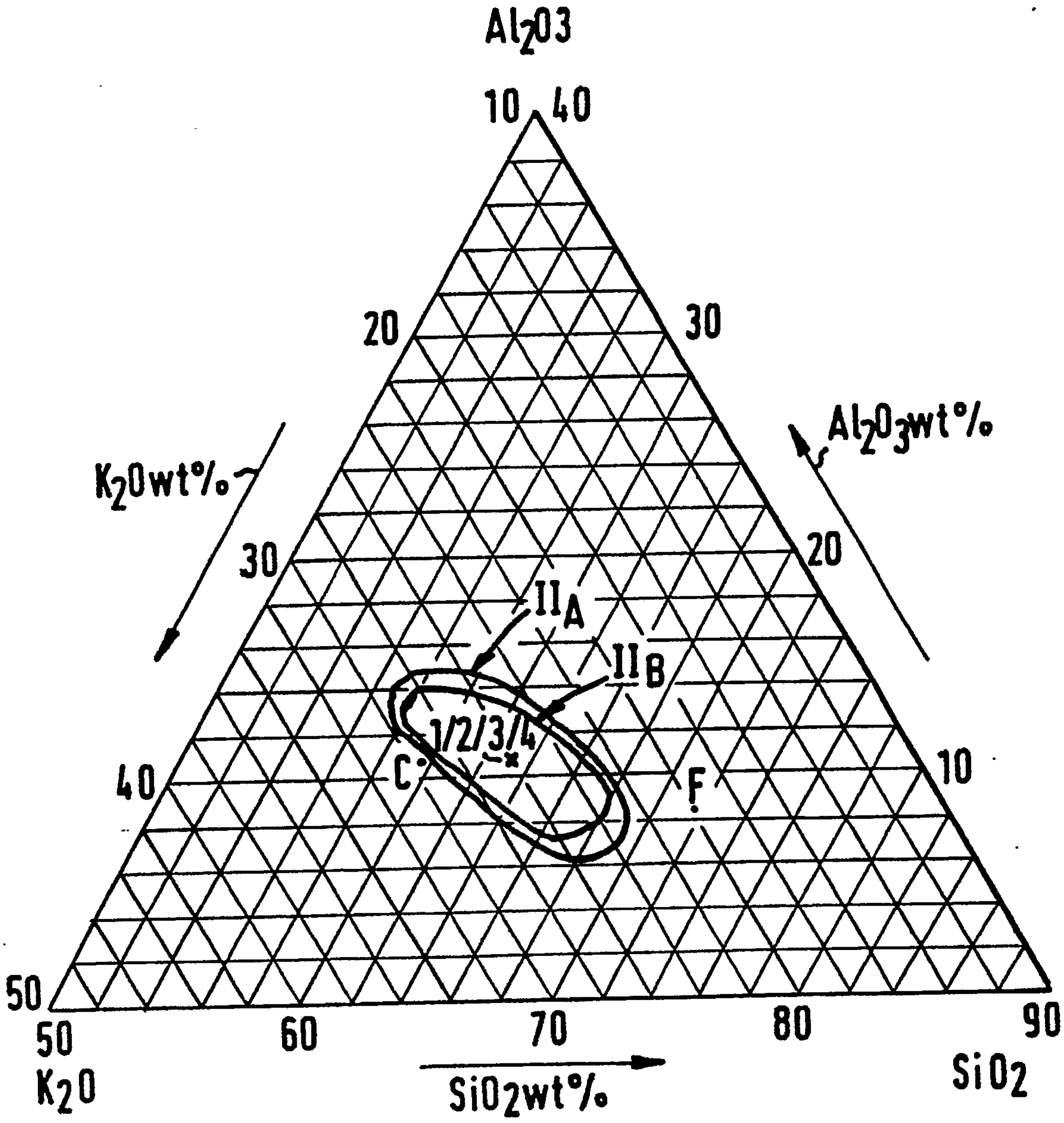
Al2O3
10
40
20
30
K2O wt%
Al2O3 wt%
30
20
II A
II B
1/2/3/4
C
F
40
10
50
50
60
70
80
90
K2O
SiO2 wt%
SiO2

FIG. 2

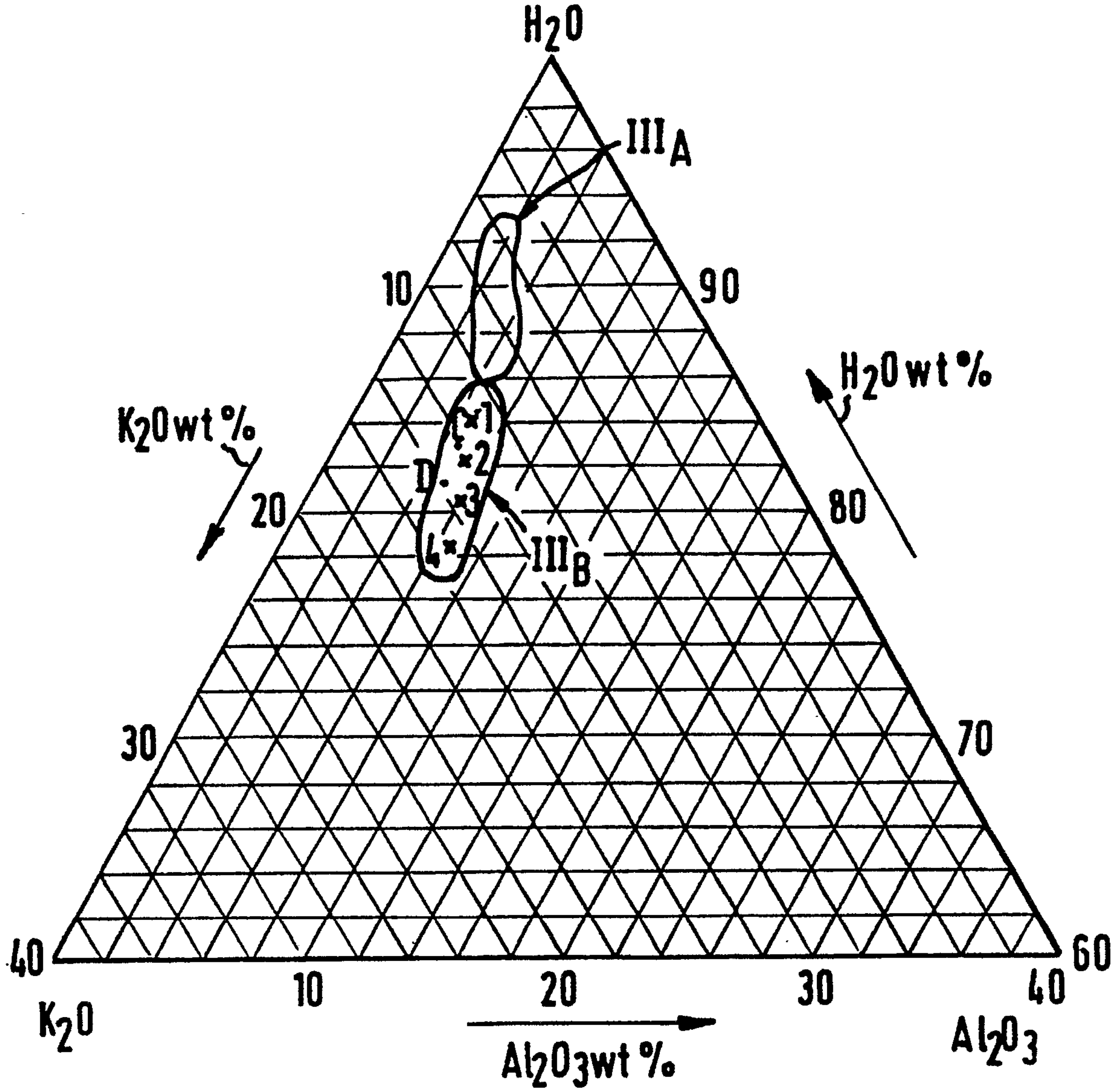
H2O
IIIA
10
90
H2O wt %
K2O wt %
1
2
D
3
20
80
4
IIIB
30
70
40
60
K2O
10
20
30
40
Al2O3
Al2O3 wt %

FIG.3

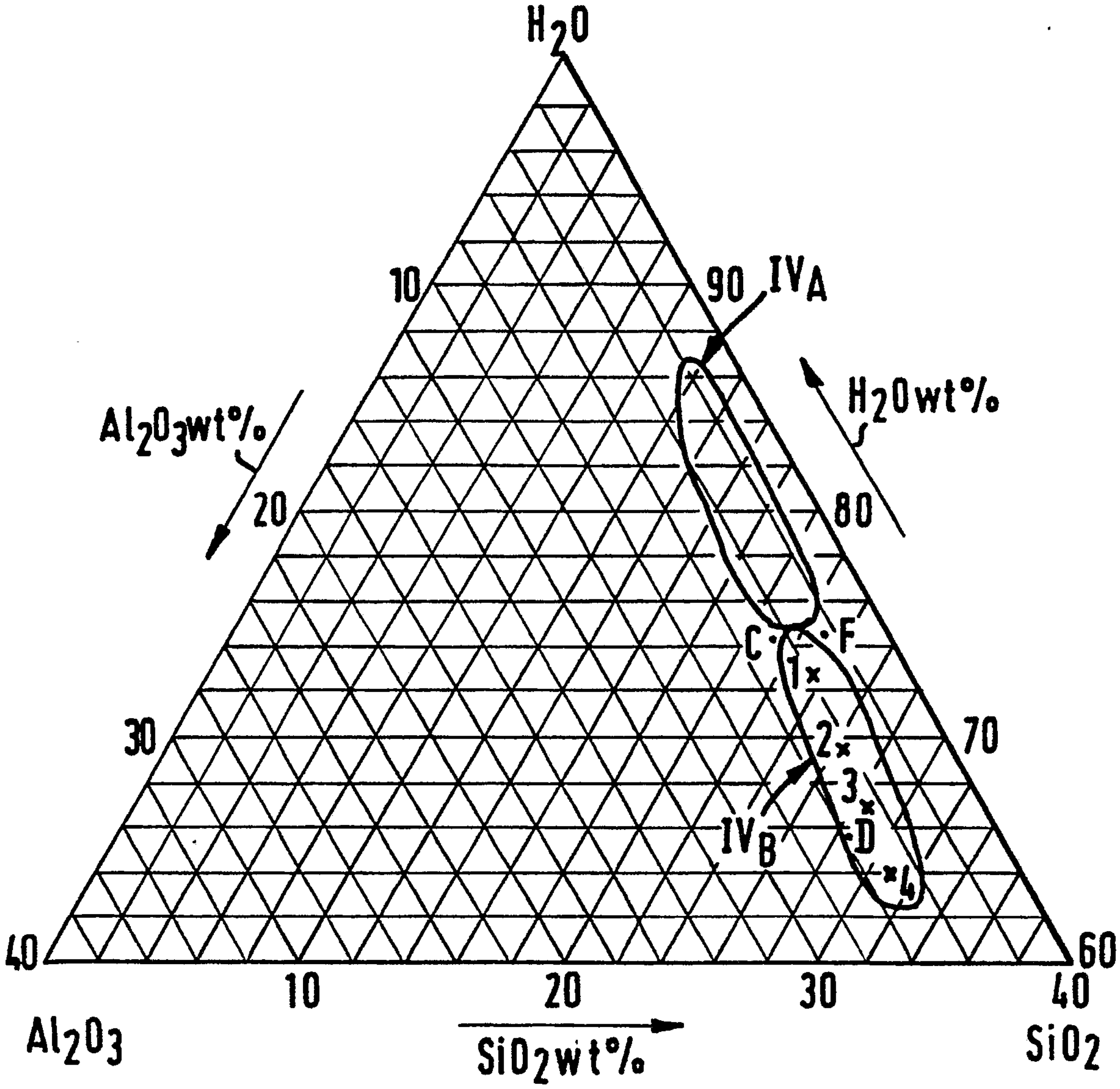
H2O
10
90
IVA
H2O wt%
Al2O3 wt%
20
80
C
F
1
30
2
70
3
IVB
D
4
40
60
10
20
30
40
Al2O3
SiO2 wt%
SiO2

FIG.4